# FASCICULE DE BREVET EUROPEEN

(11) **EP 1 001 743 B1**
(45) Date de publication et mention de la délivrance du brevet: **01.02.2006**
(21) Numéro de dépôt: 98930779.8
(22) Date de dépôt: 02.06.1998
(51) Int. Cl.: A61K 9/00

(54) **IMPLANTS POUR LA LIBERATION CONTROLEE DE PRINCIPES PHARMACEUTIQUEMENT ACTIFS ET PROCEDE POUR LEUR FABRICATION**
IMPLANTATE ZUR GESTEUERTEN FREISETZUNG VON PHARMAZEUTISCHEN WIRKSTOFFEN UND VERFAHREN ZUR HERSTELLUNG
IMPLANTS FOR CONTROLLED RELEASE OF PHARMACEUTICALLY ACTIVE PRINCIPLES AND METHOD FOR MAKING SAME

(30) Priorité: 04.06.1997 FR 9706874
(43) Date de publication de la demande: 24.05.2000
(73) Titulaire: DEBIO RECHERCHE PHARMACEUTIQUE S.A., CH-1920 Martigny (CH)
(72) Inventeur: ROTHEN-WEINHOLD, Alexandra, CH-1227 Carouge (CH); GURNY, Robert, CH-1204 Genève (CH); ORSOLINI, Piero, CH-1920 Martigny (CH); HEIMGARTNER, Frédéric, CH-1844 Villeneuve (CH)
(74) Mandataire: Nargolwalla, Cyra
(86) Numéro de dépôt international: PCT/EP1998/003270
(87) Numéro de publication internationale: WO 1998/055101

(56) Documents cités:
- WO-A-91/11176
- WO-A-92/14450
- A. ROTHEN-WEINHOLD: "Development of a long-term delivery system for RC-160, a somatostatin analog" THE 24TH INTERNATIONAL SYMPOSIUM ON CONTROLLED RELEASE OF BIOACTIVE MATERIALS, vol. 24, 15 - 19 juin 1997, pages 827-828, XP002055081 STOCKHOLM (SE)

## Description

La présente invention concerne un implant pour la libération contrôlée de principes pharmaceutiquement actifs. Elle concerne également un procédé pour la fabrication d'un tel implant.

Les formulations de médicaments à libération contrôlée sont extrêmement utiles pour l'administration de produits médicaux et pharmaceutiques et trouvent une grande variété d'applications dans lesquelles elles offrent de nombreux avantages par rapport aux formulations de médicaments standards.

Une simple administration d'une formulation à libération contrôlée assure une libération lente du principe actif au cours d'une durée prolongée.

Une des premières applications de ce type de formulation à libération contrôlée s'est située dans le domaine de la toxicomanie. Le traitement de patients qui s'adonnent à l'usage des drogues est rendu délicat et difficile dans la mesure où, avec des formulations standards, il n'est pas toujours facile d'obtenir la coopération du patient. Ainsi, avec les formulations standards, il existe toujours le risque que le patient refuse de suivre le traitement nécessaire au moment voulu. Avec les formulations à libération contrôlée, une simple administration assure par contre un traitement effectif pour une certaine durée, supérieure à ce que l'on peut obtenir à partir d'une dose unique.

Les formulations de médicaments à libération contrôlée sont également particulièrement utiles dans des applications telles que la thérapie anti-cancéreuse où des traitements à long-terme sont souvent nécessaires.

Une autre application importante de ces formulations se situe dans le domaine de la thérapie hormonale, par exemple concernant les contraceptifs, où une libération continue du principe actif à une concentration relativement constante est nécessaire pendant une certaine durée.

Les formulations de médicaments à libération contrôlée peuvent être proposées dans une variété de formes galéniques. Ainsi, il existe des formulations d'implants, ainsi que des formulations permettant l'administration orale ou parentérale.

Les formulations orales se présentent normalement sous la forme de comprimés ou de gélules pouvant être facilement avalées ou ingérées.

Les formulations parentérales ont souvent une structure sphérique. Elles souffrent d'une contrainte de taille car elles doivent pouvoir être introduites dans le corps du patient par injection au moyen d'une aiguille d'un diamètre raisonnable. Ces formulations sont souvent fabriquées à l'aide de procédés de granulation ou de microencapsulation.

Les implants peuvent se présenter sous plusieurs formes. Ils sont ainsi souvent moulés sous la forme de films ou de pellicules puis traités pour donner des formes différentes, telles que des bâtonnets cylindriques, des particules sphériques et autres.

Selon le but et les circonstances de la forme souhaitée de thérapie, les implants peuvent parfois être préférés aux formulations parentérales. Cette préférence existe notamment dans le cas où l'on souhaite une certaine flexibilité par rapport au protocole thérapeutique. Par rapport aux formulations parentérales, les implants présentent l'avantage de pouvoir être enlevés chirurgicalement, s'il s'avère nécessaire d'arrêter le traitement avant que n'ait eu lieu la libération complète du principe actif.

Un phénomène souvent observé avec les formulations de médicaments à libération contrôlée est celui du "burst effect", c'est-à-dire une libération initiale très importante du principe actif. Dans certains cas, cet effet peut être souhaité. En revanche, il existe des cas où il peut se révéler dangereux. Ceci est le cas notamment en ce qui concerne les thérapies hormonales qui utilisent des principes actifs ayant des effets secondaires très gênants, voire toxiques, à des concentrations importantes. Dans de tels cas, il est impératif de pouvoir assurer une libération lente et uniforme, par petites quantités, du principe actif.

La demande de brevet EP 0 659 406 aux noms de DOW CORNING ASIA Ltd. et SUMITOMO PHARMACEUTICALS COMPANY Ltd. fournit une solution possible au problème décrit ci-dessus. Cette demande décrit un bâtonnet cylindrique de formulation à libération contrôlée comprenant une couche interne qui ne se désagrège pas et une couche externe imperméable à l'eau. Dans les exemples de cette demande, on peut lire que cette formulation est préparée par immersion de la couche interne dans une solution contenant la substance qui constitue la couche externe ou le revêtement. Cette méthode dite de "trempage" ou d'immersion est également décrite dans le brevet US 4,894,231 au nom de la Société BIOMEASURE, Inc. Dans ce brevet, la couche externe est également une substance imperméable à l'eau qui sert de "barrière", évitant ainsi une libération initiale trop importante du principe actif.

Ce brevet, outre la méthode de trempage, décrit également le revêtement de la couche interne par une couche externe à l'aide d'une pulvérisation.

Tous les implants ou autres formulations de médicaments à libération contrôlée fabriqués par les méthodes décrites ci-dessus souffrent cependant de plusieurs défauts.

La constitution de la couche externe par les méthodes telles que le trempage ou la pulvérisation rend très difficile et onéreuse l'obtention d'une couche de surface lisse et uniforme. Ces méthodes présentent également l'inconvénient de ne pas permettre un contrôle fiable de l'épaisseur de la couche. Or, il serait très souhaitable de pouvoir obtenir, par une méthode simple, des implants comportant une couche externe lisse et uniforme, d'une épaisseur choisie, ce qui permettrait également de mieux contrôler voire limiter le "burst effect".

Les méthodes dites de trempage présentent un inconvénient supplémentaire. En effet, ces méthodes nécessitent l'utilisation de solvants organiques tels que l'acétone, le chloroforme ou le chlorure de méthylène. Il est donc par la suite nécessaire d'enlever ces solvants afin de pouvoir introduire les implants ou autres formulations dans le corps du sujet à traiter.

WO-A-92/14450 décrit une méthode et un procédé pour la libération contrôlée d'un principe actif chez un mammifère. Le dispositif décrit comprend un noyau ou un réservoir renfermant le principe actif, une première couche, d'enrobage substantiellement imperméable au passage de l'agent actif et une seconde couche d'enrobage perméable au passage de l'agent actif. La première couche couvre au moins une partie du noyau, mais au moins une petite portion du noyau n'est pas enrobée avec ladite première couche. La seconde couche couvre, complètement la première couche d'enrobage et la partie du noyau qui n'est pas enrobée. La première couche est de préférence réalisée en éthylène vinyl-acétate et la seconde couche d'enrobage en alcool polyvinylique.

WO-A-91/11176 décrit un article pour la libération contrôlée d'un principe actif comprenant un espace creux totalement enfermé par une paroi et rempli en tout ou partie d'un ou plusieurs principes actifs. Ladite paroi est réalisée en matériau polymérique biodégradable perméable au principe actif, tandis que la paroi est principalement composée d'une combinaison d'au moins deux matériaux polymères différents, où un matériau polymérique est perméable au principe actif et est dégradable, et l'autre matériau polymérique est relativement peu perméable au principe actif et est également dégradable.

*La Société Demanderesse, après de nombreux travaux et recherches, est parvenue à répondre au besoin existant d'un implant pour la libération contrôlée des principes pharmaceutiquement actifs pour lequel le "burst effect" est sensiblement contrôlé, tout en évitant les inconvénients des formulations décrites dans l'art antérieur.

L'invention a donc pour objet un implant pour la libération contrôlée d'au moins un principe pharmaceutiquement actif, cet implant comprenant un noyau qui contient au moins un principe actif et une gaine qui entoure ledit noyau, et il est caractérisé par le fait que ladite gaine est constituée d'au moins un film polymériques défini ci-après appliqué autour dudit noyau.

Selon un mode préférentiel de réalisation de l'invention, la gaine est constituée d'au moins deux films polymériques, l'un entourant une partie du noyau et l'autre entourant la partie restante.

La gaine consiste en au moins un film polymérique appliqué autour du noyau. Ainsi, on peut avoir une gaine consistant en :
- au moins deux films polymériques de nature identique ou différente qui sont appliqués chacun autour d'une partie du noyau comme indiqué ci-dessus,
- ou bien, au moins deux films polymériques de nature identique ou différente superposés, appliqués autour du noyau.

Bien entendu, la gaine peut également consister en les deux films polymériques juxtaposés pour entourer la totalité du noyau, sur lesquels est appliqué au moins un autre film polymérique.

L'implant selon l'invention présente l'avantage de posséder une gaine parfaitement lisse et uniforme dont l'épaisseur peut être choisie, ce qui permet une grande flexibilité en ce qui concerne le choix du profil de libération, offrant ainsi la possibilité d'une régulation plus précise du "burst effect" et ainsi de la vitesse de libération initiale du principe pharmaceutiquement actif. Cette épaisseur peut être contrôlée en fonction de l'épaisseur du film polymérique choisi et du nombre de films polymériques superposés, appliqués autour du noyau.

Avantageusement, le noyau est constitué d'un polymère biodégradable, mélangé avec au moins un principe pharmaceutiquement actif.

Le polymère de la gaine peut éventuellement être préalablement mélangé avec un ou plusieurs principes pharmaceutiquement actifs. Ces principes actifs peuvent éventuellement être les mêmes que ceux qui sont associés avec le polymère constituant le noyau.

Les polymères biodégradables utilisés dans l'implant conforme à l'invention peuvent être choisis parmi le groupe comprenant : les polycaprolactones, les polyorthoesters, les polyanhydrides, les polyacétals et les acides α-hydroxy-carboxyliques, tels que les homopolymères et copolymères de l'acide lactique, de l'acide glycolique et de l'acide succinique.

En effet, la découverte de polymères facilement biodégradables a fait considérablement progresser la technologie du domaine des formulations médicamenteuses à libération retardée. Il est bien entendu avantageux de pouvoir introduire une formulation médicamenteuse polymère dans le corps humain ou animal en sachant que le polymère va se dégrader au cours d'un certain laps de temps, autorisant une lente libération du médicament sans laisser subsister de matières étrangères dans le corps du patient.

En ce qui concerne les polymères biodégradables, les copolymères et homopolymères des acides lactiques et glycoliques sont particulièrement préférés dans les implants à libération contrôlée selon l'invention. Ils sont facilement dégradables, se décomposant en produits inoffensifs tels que le dioxyde de carbone et l'eau, et ne laissent donc aucun résidu au cours du temps après la libération du principe actif.

Ces polymères seront ici communément appelés les "polylactides".

Dans le contexte de la présente invention, le terme "polylactides" vise à inclure tant sa signification générique, c'est-à-dire un polyester dérivé d'un acide α-hydroxycarboxylique, que sa signification spécifique, c'est-à-dire un polymère dérivé de l'acide lactique (acide α-hydroxypropionique). Le terme est de plus censé englober des copolymères des acides glycoliques et lactiques ainsi que des homopolymères de l'un ou l'autre de ces acides.

Les polylactides avantageusement utilisés pour constituer le noyau de l'implant sont les acides L-polylactiques de bas poids moléculaire. Ces polymères ont un poids moléculaire situé entre 2000 et 30000, de préférence entre 2000 et 15000, et plus préférentiellement encore entre 4000 et 6000, plus préférentiellement de 5000 daltons.

La gaine de l'implant conforme à l'invention est constituée d'un mélange d'un polymère de l'acide polylactide-co-glycolide (PLGA) et d'un polymère de l'acide polylactide (PLA). De préférence, le mélange est réalisé dans des proportions 40:60 à 60 :40 et plus préférentiellement encore 50:50. Le poids moléculaire du PLGA utilisé se situe avantageusement entre 20.000 et 100.000 daltons, de préférence entre 40.000 et 60.000 daltons et plus préférentiellement encore est de 55.000 daltons. Le poids moléculaire de l'acide polylactide utilisé se situe de préférence entre 2.000 et 6.000 daltons, et plus préférentiellement encore est de 5.000 daltons.

Cependant, après de nombreuses recherches, la Société Demanderesse a pu constater que le polymère consistant en un mélange spécifique d'un acide poly(lactide-co-glycolide) dans des proportions de 50/50 en poids, et ayant un poids moléculaire de 55.000 daltons, et d'un acide polylactide ayant un poids moléculaire de 5.000 daltons, permet l'obtention d'une gaine qui est particulièrement bien adaptée à la libération contrôlée d'un principe pharmaceutiquement actif.

En effet, le polymère d'acide poly(lactide-co-glycolide) cité ci-dessus possède, à lui seul, un certain nombre d'avantages tel que l'obtention de films homogènes ayant un débit de dégradation quasi-immédiat. Cependant, il souffre de l'inconvénient que les films obtenus sont très cassants et donc non adaptés à l'utilisation en tant que gaine.

Quant au polymère d'acide polylactide, il donne des films qui ne sont pas cassants. En revanche, ces films ne sont pas homogène et leur vitesse de dégradation n'est pas très rapide.

La Société Demanderesse a constaté, contre toute attente, que la combinaison de ces deux polymères permettrait l'obtention de films homogènes, non cassants, et possédant une vitesse de dégradation parfaitement adaptée pour constituer la gaine d'un implant pour la libération d'un principe pharmaceutiquement actif.

Ainsi, l'invention concerne également un polymère destiné à constituer la gaine d'un implant pour la libération contrôlée d'au moins un principe pharmaceutiquement actif.

Ce polymère est constitué d'un mélange :
- d'un acide poly(lactide-co-glycolide) ayant un poids moléculaire situé entre 20.000 et 100.000 daltons, de préférence entre 40.000 et 60.000 daltons, et plus préférentiellement encore de 55.000 daltons, et
- d'un acide polylactide ayant un poids moléculaire situé entre 2.000 et 6.000 daltons, de préférence de 5.000 daltons.

De façon avantageuse, le rapport en poids des deux constituants du mélange est compris entre 40:60 et 60:40, étant de préférence de 50:50.

Les implants conformes à la présente invention permettent, grâce à la sélection judicieuse des polymères qui constituent le noyau et la gaine, d'ajuster le temps de libération ainsi que le profil de libération d'un principe actif.

Ainsi, selon les interactions hydrophiles /hydrophobes entre les polymères et le ou les principes actifs, on peut obtenir une libération plus ou moins rapide ou plus ou moins lente.

De surcroît, il est possible de modifier le profil de libération. Par exemple, en choisissant pour la gaine un polymère dégradable très rapidement, il est possible d'obtenir une libération immédiate importante. Toutefois, si on choisit un polymère pour la gaine qui n'est pas facilement dégradable, cette libération initiale est atténuée.

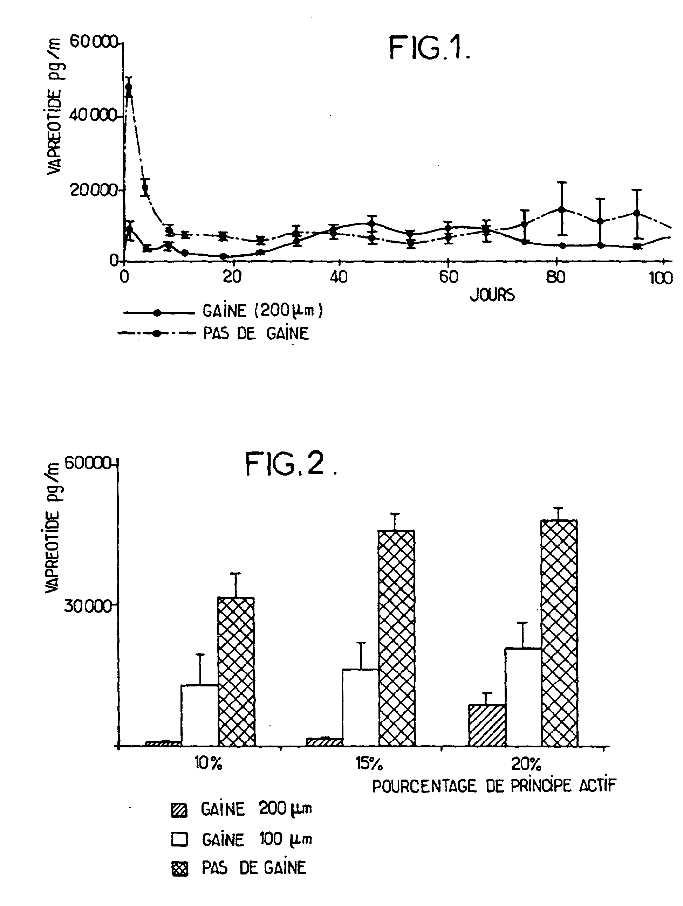

La Société Demanderesse a constaté de façon surprenant et inattendue, que les implants selon l'invention permettent une libération régulière du principe actif, même après disparition de la gaine. En effet, on aurait pu s'attendre à ce qu'il y ait un "burst effect" après la dissolution de la gaine mais ceci ne se produit pas. La Figure 1 montre que le "burst effect" initial est presque entièrement éliminé pour les implants selon l'invention par comparaison avec des implants constitués seulement d'un noyau, et, de surcroît, qu'il n'y a pas de "burst effect" retardé.

La Figure 2 montre l'effet de l'épaisseur de la gaine sur le "burst effect". La libération initiale de principe actif est très importante pour un implant ne comportant pas de gaine en comparaison avec des implants selon l'invention ayant des gaines d'une épaisseur de 100 µm ou de 200 µm.

Selon la présente invention, on peut également établir des protocoles de libération pour plus d'un principe actif. Ainsi, si le polymère qui constitue le noyau contient un principe actif et le polymère de la gaine contient également un principe actif, identique ou différent, il est possible d'obtenir des protocoles de libération complexes, dans lesquels le ou les principes actifs peuvent être libérés simultanément ou consécutivement.

Ainsi, la structure des implants selon l'invention offre une plus grande souplesse dans la sélection des vitesses et des profils de libération pour les différents types de principes actifs.

Parmi la grande variété de principes actifs pouvant être utilisés dans le cadre de l'invention, on peut citer les hormones, les enzymes, les polypeptides, les protéines, les vaccins, les antibiotiques, les substances anticancéreuses et les autres substances bioactives.

Selon un mode de réalisation particulier de l'invention, le principe actif est un analogue de la Somatostatine ou l'un de ses sels pharmaceutiquement acceptables. De préférence, ce principe actif est le pamoate de Vapréotide.

L'invention a également pour objet un procédé pour la fabrication des implants pour la libération contrôlée d'au moins un principe actif.

Ce procédé est caractérisé par le fait que :
- l'on fabrique un noyau contenant au moins un principe actif,
- l'on prépare au moins un film polymérique,
- l'on applique le ou lesdits films polymériques autour dudit noyau, formant ainsi une gaine,
- l'on soumet l'implant ainsi obtenu à une stérilisation.

La fabrication du noyau peut être effectuée par toute méthode connue de l'homme du métier, telle que l'extrusion ou par moulage. De façon préférentielle, on prépare un mélange du polymère avec le principe actif, on homogénéise le mélange et on effectue une extrusion à une température choisie en fonction de la nature du polymère et du principe actif.

Le film polymérique est préparé par toute méthode connue. De préférence, on chauffe le ou les polymères choisis et on soumet le mélange à une pression hydraulique à une température choisie en fonction de la nature des polymères.

On peut soumettre le film polymérique à une étape intermédiaire de moulage par laquelle l'épaisseur du film est mieux contrôlée. Pour ce faire, on met le film dans une partie "femelle", concave d'un moule, on pose une partie "mâle", convexe dessus, de sorte qu'il existe un espace de l'épaisseur voulue entre les deux parties du moule. Ensuite, on exerce une pression.

Le film polymérique est appliqué sur le noyau, par exemple en l'enroulant autour du noyau. Ou bien encore, des films polymériques sont disposés dans les deux parties creuses d'un moule à deux coques, à l'intérieur duquel est disposé le noyau.

La stérilisation des implants obtenus conformément à l'invention est effectuée par irradiation avec des rayons gamma.

Selon un mode préférentiel de réalisation du procédé de l'invention, on applique successivement chacun des films polymériques au noyau.

Selon un autre mode de réalisation du procédé conforme à l'invention, on applique un film polymérique sur une partie du noyau, et on applique un deuxième film polymérique sur la partie restante du noyau.

Le noyau est avantageusement constitué d'un polymère biodégradable, ce polymère contenant au moins un principe actif.

Le ou les films polymériques de la gaine peuvent également être constitués de polymères biodégradables.

Les polymères biodégradables du noyau et/ou du film polymérique de la gaine peuvent être choisis parmi le groupe comprenant les polycaprolactones, les polyorthoesters et les acides α-hydroxy-carboxyliques, de préférence les homopolymères et copolymères de l'acide l'actique, de l'acide glycolique et de l'acide succinique.

De façon préférentielle, le film polymérique de la gaine est constitué d'un mélange, dans des proportions de 40:60 à 60:40, de préférence de 50:50, d'un acide poly(lactique-co-glycolide) ayant un poids moléculaire situé entre 20.000 et 100.000 daltons, de préférence entre 40.000 et 60.000 daltons et plus préférentiellement encore étant de 55.000 daltons, et d'un acide polylactide ayant un poids moléculaire situé de préférence entre 2.000 et 6.000 daltons, et plus préférentiellement encore étant de 5.000 daltons.

Selon un mode de réalisation préférentielle, le principe actif utilisé dans le procédé selon l'invention est un analogue de la Somatostatine ou de l'un de ses sels pharmaceutiquement acceptables, de préférence le pamoate de Vapréotide.

### Exemple 1 Fabrication d'un implant selon l'invention

Le procédé de fabrication des implants selon l'invention comporte les cinq étapes suivantes :
1) fabrication du noyau,
2) fabrication du film d'enrobage,
3)moulage des films polymériques d'enrobage,
4)mise en place de la gaine autour du noyau,
5)stérilisation.

### 1) Fabrication du noyau

On mélange 3 g d'un polymère de l'acide L-lactique (appelé L104 et commercialisé par la Société Boehringer Ingelheim, Ingelheim am Rhein, Allemagne) d'un poids moléculaire d'environ 5.000 daltons avec 857 mg du principe actif dénommé RC 160, qui est un pamoate de Vapréotide, un analogue de la somatostatine, commercialisé par la Société Nova Biochem, Suisse, à l'aide d'un mélangeur tridimensionnel.

On homogénéise le mélange dans un mortier en agate, puis on le déionise.

On effectue l'extrusion du mélange obtenu à une température d'environ 80° C à l'aide d'une extrudeuse à piston de diamètre de filière 1,5 mm.

On coupe l'extrudat ainsi obtenu en bâtonnets de 15 mm de longueur.

### 2) Fabrication du film d'enrobage

On mélange 1 g du polymère RG 504 (commercialisé par la société Boehringer Ingelheim) qui est un polymère poly(lactide-co-glycolide) mélange 50:50 ayant un poids moléculaire de 55.000 daltons, et 1 g du polymère L104 (L-PLA) (commercialisé par Boehringer Ingelheim) ayant un poids moléculaire de 5.000 daltons.

Le mélange est ensuite malaxé sur des cylindres qui roulent sur eux-mêmes, chauffés à 80° C afin d'homogénéiser le mélange.

On place le mélange pendant 30 secondes entre deux feuilles anti-adhérentes dans une presse hydraulique, à une température de 75° C en exerçant une pression de 30 bars.

Le film ainsi obtenu est transparent et homogène, et présente une épaisseur d'environ 200 µm.

### 3) Moulage des films polymériques d'enrobage

Le film obtenu est coupé en deux parties. Chaque partie est appliquée sur la partie "femelle", concave, d'un moule revêtu de Téflon® à une température de 65° C. Une empreinte constituant la partie "mâle" convexe dudit moule est posée sur le film. L'ensemble est placé sous presse à 65° C pendant 30 secondes, temps qui correspond au ramollissement du film et à sa mise en forme dans le moule.

L'épaisseur de la gaine obtenue est fonction de l'espace qui existe entre les deux parties du moule.

### 4) Mise en place de la gaine autour du noyau

On laisse les deux films polymériques dans leurs moules respectifs. On dispose sur l'un d'eux un noyau cylindrique d'une longueur de 15 mm et d'un diamètre de 1,5 mm. On place au-dessus l'autre film polymérique maintenu dans son moule. On met l'ensemble sous presse à 65°C pendant une minute. On démoule lorsque l'intérieur du moule atteint 50°C, obtenant ainsi un implant selon l'invention.

### 5) Stérilisation de l'implant

L'implant ainsi obtenu est ensuite stérilisé par irradiation à 25 kGy à une température de -78° C avec des rayons γ (source ⁶⁰Co) à une vitesse d'environ 0,797 kGy/h.

### 6) Etude in-vivo des effets des implants

Des implants obtenus selon la méthode indiquée ci-dessus ont été placés, par voie sous-cutanée, sur le dos des rats Sprague Dawley ayant un poids moyen de 330 à 340g.

Des échantillons de sans ont été prélevés de 24 heures jusqu'à 260 jours et les taux de principe actif ont été déterminés par des radio-immuno essais utilisant un anticorps double.

Les résultats sont présentés sur les Figures 1 et 2.

## Revendications

1. Polymère destiné à constituer la gaine d'un implant pour la libération contrôlée d'un principe pharmaceutiquement actif, **caractérisé par le fait qu'**il est constitué :
- d'un acide poly(lactide-co-glycolide), ayant un poids moléculaire situé entre 20.000 et 100.000 daltons, de préférence entre 40.000 et 60.000 daltons, et plus préférentiellement encore étant de 55.000 daltons, et
- d'un acide polylactide ayant un poids moléculaire situé entre 2.000 et 6.000 daltons, de préférence étant de 5.000 daltons.

2. Polymère selon la revendication 1, **caractérisé par le fait que** le rapport en poids de l'acide poly(lactide-co-glycolide) et de l'acide polylactide est compris entre 40:60 et 60:40, de préférence de 50:50.

3. Implant pour la libération contrôlée d'au moins un principe pharmaceutiquement actif comprenant :
- un noyau contenant au moins un principe actif, et
- une gaine entourant ledit noyau,
**caractérisé par le fait que** ladite gaine est constituée d'au moins un film polymérique constitué :
- d'un acide poly(lactide-co-glycolide), ayant un poids moléculaire situé entre 20.000 et 100.000 daltons, de préférence entre 40.000 et 60.000 daltons, et plus préférentiellement encore étant de 55.000 daltons, et
- d'un acide polylactide ayant un poids moléculaire situé entre 2.000 et 6.000 daltons, de préférence étant de 5.000 daltons,
appliqué autour dudit noyau.

4. Implant selon la revendication 3, **caractérisé par le fait que** la gaine est constituée d'au moins deux films polymériques, l'un entourant une partie du noyau et l'autre entourant la partie restante, sur ces deux films peut éventuellement être superposé(s) au moins un autre film(s) polymérique(s).

5. Implant pour la libération contrôlée d'au moins un principe pharmaceutiquement actif selon l'une ou l'autre des revendications 3 et 4, **caractérisé par le fait que** le noyau est constitué d'un polymère biodégradable contenant au moins un principe actif.

6. Implant pour la libération contrôlée d'au moins un principe pharmaceutiquement actif selon l'une quelconque des revendications 3 à 5, **caractérisé par le fait qu'**au moins un film polymérique constituant la gaine contient au moins un principe actif.

7. Implant pour la libération contrôlée d'au moins un principe pharmaceutiquement actif selon l'une quelconque des revendications 5 et 6, **caractérisé par le fait que** le polymère biodégradable du noyau est choisi parmi le groupe comprenant les polycaprolactones, les polyorthoesters et les acides α-hydroxy-carboxyliques, de préférence les homopolymères et copolymères de l'acide lactique, de l'acide glycolique, et de l'acide succinique.

8. Implant pour la libération contrôlée d'un principe pharmaceutiquement actif selon l'une quelconque des revendications 5 et 6, **caractérisé par le fait que** le film polymérique de la gaine est constitué d'un mélange, dans des proportions de 40:60 à 60:40, de préférence de 50:50, de l'acide poly(lactide-co-glycolide) et de l'acide polylactide.

9. Implant pour la libération contrôlée d'au moins un principe pharmaceutiquement actif selon l'une quelconque des revendications 3 à 8, **caractérisé par le fait que** le principe actif est un analogue de la Somatostatine ou de l'un de ses sels pharmaceutiquement acceptables, ou de préférence le pamoate de vapréotide.

10. Procédé de préparation d'un implant pour la libération contrôlée d'au moins un principe pharmaceutiquement actif, tel que défini à l'une quelconque des revendications 3 à 9, **caractérisé par le fait :**
- **que** l'on fabrique un noyau contenant au moins un principe actif,
- **que** l'on prépare au moins un film polymérique, à partir d'un polymère tel que défini à la revendication 1 ou 2,
- **que** l'on applique le ou lesdits films polymériques autour dudit noyau, en les juxtaposant et/ou en les superposant,
- et **que** l'on soumet l'implant ainsi obtenu à une stérilisation.

## Patentansprüche

1. Polymer zur Bildung der Umhüllung eines Implantats zur gesteuerten Freisetzung eines pharmazeutischen Wirkstoffs, **dadurch gekennzeichnet, dass** es gebildet wird aus:
- einem Polylactid-co-glycolid mit einem Molekulargewicht zwischen 20.000 und 100.000 D, vorzugsweise zwischen 40.000 und 60.000 D, und weiter bevorzugt von 55.000 D, und
- einer Polymilchsäure mit einem Molekulargewicht zwischen 2.000 und 6.000 D, vorzugsweise von 5.000 D.

2. Polymer gemäß Anspruch 1, **dadurch gekennzeichnet, dass** das Gewichtsverhältnis von Polylactid-co-glycolid zu Polymilchsäure zwischen 40:60 und 60:40 ist, vorzugsweise 50:50.

3. Implantat zur gesteuerten Freisetzung von mindestens einem pharmazeutischen Wirkstoff, das folgendes umfasst:
- einen Kern, der mindestens einen Wirkstoff enthält, und
- eine Umhüllung, die diesen Kern umgibt,
**dadurch gekennzeichnet, dass** die Umhüllung aufgebaut ist aus mindestens einem Polymerfilm, der gebildet ist aus:
- einem Polylactid-co-glycolid mit einem Molekulargewicht zwischen 20.000 und 100.000 D, vorzugsweise zwischen 40.000 und 60.000 D, und weiter bevorzugt von 55.000 D, und
- einer Polymilchsäure mit einem Molekulargewicht zwischen 2.000 und 6.000 D, vorzugsweise von 5.000 D,
und der um den Kern herum aufgebracht ist.

4. Implantat gemäß Anspruch 3, **dadurch gekennzeichnet, dass** die Umhüllung aus mindestens zwei Polymerfilmen gebildet wird, wobei der eine einen Teil des Kerns und der andere den verbleibenden Teil umgibt und über diese beiden Filme ggf. mindestens ein weiterer Polymerfilm aufgebracht ist.

5. Implantat zur gesteuerten Freisetzung mindestens eines pharmazeutischen Wirkstoffes gemäß mindestens einem der Ansprüche 3 und 4, **dadurch gekennzeichnet, dass** der Kern aus einem biologisch abbaubaren Polymer aufgebaut ist, das mindestens einen Wirkstoff enthält.

6. Implantat zur gesteuerten Freisetzung mindestens eines pharmazeutischen Wirkstoffes gemäß mindestens einem der Ansprüche 3 bis 5, **dadurch gekennzeichnet, dass** mindestens ein Polymerfilm, aus dem die Umhüllung gebildet wird, mindestens einen Wirkstoff enthält.

7. Implantat zur gesteuerten Freisetzung mindestens eines pharmazeutischen Wirkstoffes gemäß mindestens einem der Ansprüche 5 und 6, **dadurch gekennzeichnet, dass** das biologisch abbaubare Polymer des Kerns ausgewählt ist aus Polycaprolactonen, Polyorthoestern und α-Hydroxycarbonsäuren, vorzugsweise aus Homopolymeren und Copolymeren von Milchsäure, Glykolsäure und Bernsteinsäure.

8. Implantat zur gesteuerten Freisetzung mindestens eines pharmazeutischen Wirkstoffes gemäß mindestens einem der Ansprüche 5 und 6, **dadurch gekennzeichnet, dass** der Polymerfilm der Umhüllung aus einer Mischung aus Polylactid-co-glykolid und Polymilchsäure in einem Verhältnis von 40:60 bis 60:40, vorzugsweise von 50:50, gebildet wird.

9. Implantat zur gesteuerten Freisetzung mindestens eines pharmazeutischen Wirkstoffes gemäß mindestens einem der Ansprüche 3 bis 8, **dadurch gekennzeichnet, dass** der Wirkstoff ein Analagon von Somatostatine oder eines pharmazeutisch annehmbaren Salzes davon, oder vorzugsweise Vapreotidpamoat ist.

10. Verfahren zur Herstellung eines Implantats zur gesteuerten Freisetzung mindestens eines pharmazeutischen Wirkstoffes wie in mindestens einem der Ansprüche 3-9 definiert, das **gekennzeichnet ist durch**:
- Herstellen eines Kerns, der mindestens einen Wirkstoff enthält,
- Herstellen mindestens eines Polymerfilms aus einem Polymer wie in Anspruch 1 oder 2 definiert,
- Aufbringen des oder der Polymerfilm(s/e) um den Kern in nebeneinander und/oder übereinander liegender Weise, und
- Sterilisieren des so erhaltenen Implantats.

## Claims

1. Polymer intended to form the sheath of an implant for the controlled release of a pharmaceutically active principle, consisting of:
- a poly(lactide-co-glycolide) acid having a molecular weight in the range 20 000 daltons to 100 000 daltons, preferably in the range 40 000 daltons to 60 000 daltons and even more preferably 55 000 daltons, and
- a polylactide acid having a molecular weight in the range 2 000 daltons to 6 000 daltons, and preferably 5 000 daltons.

2. Polymer according to claim 1, wherein the weight ratio of poly(lactide-co-glycolide) acid and polylactide acid is in the range 40:60 to 60:40, preferably 50:50.

3. Implant for the controlled release of at least one pharmaceutically active principle comprising:
- a core containing at least one active principle, and
- a sheath surrounding said core,
wherein said sheath is composed of at least one polymeric film consisting of:
- a poly(lactide-co-glycolide) acid having a molecular weight in the range 20 000 daltons to 100 000 daltons, preferably in the range 40 000 daltons to 60 000 daltons and even more preferably 55 000 daltons, and
- a polylactide acid having a molecular weight in the range 2 000 daltons to 6 000 daltons, and preferably 5 000 daltons,
applied around said core.

4. Implant for the controlled release of at least one pharmaceutically active principle according to claim 3,
wherein the sheath is composed of at least two polymeric films, one surrounding a part of the core and the other surrounding the remaining part, at least one other polymeric film(s) optionally being superposed on these two films.

5. Implant for the controlled release of at least one pharmaceutically active principle according to one of claims 3 and 4, wherein the core is composed of a biodegradable polymer containing at least one active principle.

6. Implant for the controlled release of at least one pharmaceutically active principle according to one of claims 3 to 5, wherein at least one polymeric film forming the sheath contains at least one active principle.

7. Implant for the controlled release of at least one pharmaceutically active principle according to one of claims 5 and 6, wherein the biodegradable polymer of the core is selected from the group consisting of polycaprolactones, polyorthoesters and α-hydroxycarboxylic acids, preferably homopolymers and copolymers of lactic acid, glycolic acid and succinic acid.

8. Implant for the controlled release of a pharmaceutically active principle according to one of claims 5 and 6, wherein the polymeric film of the sheath is composed of a mixture, in proportions in the range 40:60 to 60:40, preferably 50:50, of the poly(lactide-co-glycolide) acid and of the polylactide acid.

9. Implant for the controlled release of at least one pharmaceutically active principle according to one of claims 3 to 8, wherein the active principle is an analogue of somatostatin or of one of the pharmaceutically acceptable salts thereof, preferably vapreotide pamoate.

10. Process for the preparation of an implant for the controlled release of at least one pharmaceutically active principle as defined one of claims 3 to 9, wherein
- a core containing at least one active principle is produced,
- at least one polymeric film is prepared from a polymer as defined in claim 1 or 2,
- the polymeric film(s) is/are applied around said core by juxtaposing and/or superposing it/them,
- the implant thus obtained is sterilised.
